# EUROPEAN PATENT APPLICATION

(11) **EP 2 078 527 A1**
(43) Date of publication of application: **15.07.2009**
(21) Application number: 07829585.4
(22) Date of filing: 11.10.2007
(51) Int. Cl.: A61K 31/496, A61K 9/08, A61K 47/10, A61K 47/36, A61P 27/02, A61P 31/04

(54) **AQUEOUS LIQUID PREPARATION COMPRISING GATIFLOXACIN**

(30) Priority: 12.10.2006 JP 2006279009
(71) Applicant: Kyorin Pharmaceutical Co., Ltd., Tokyo 101-8311 (JP); Senju Pharmaceutical Co., Ltd., Osaka-shi, Osaka 541-0046 (JP)
(72) Inventor: SAWA, Shirou, Kobe-shi Hyogo 651-2241 (JP)
(74) Representative: Gillard, Richard Edward
(86) International application number: PCT/JP2007/069847
(87) International publication number: WO 2008/044734

(57) **Abstract**

Disclosed is an aqueous liquid preparation comprising (A) gatifloxacin, a pharmacologically acceptable salt thereof or a hydrate gatifloxacin or the salt, (B) hyaluronic acid or a pharmacologically acceptable salt thereof, and (C) a polyhydric alcohol. The aqueous liquid preparation is an ophthalmic aqueous liquid preparation excellent in the retention of gatifloxacin in a tear fluid and the penetration of gatifloxacin into an aqueous humor and a conjunctiva.

## Description

### Technical Field

The present invention relates to an aqueous liquid preparation containing gatifloxacin (chemical name: (±)-1-cyclopropyl-6-fluoro-1,4-dihydro-8-methoxy-7-(3-methyl-1-piperazinyl)-4-oxo-3-quinolinecarboxylic acid). More specifically, it relates to an ophthalmic aqueous liquid preparation having improved intraocular penetration of gatifloxacin.

### Background Art

Gatifloxacin is a quinolonecarboxylic acid derivative and is a new quinolone synthetic antibacterial agent. It is recognized to exhibit strong antibacterial potency against not only Gram-negative bacteria but also Gram-positive bacteria, anaerobic bacteria, and mycoplasma, and an application to infectious diseases in ophthalmological area such as conjunctivitis, dacryoadenitis and hordeolum, and infectious diseases in otological area such as otitis externa, tympanitis and sinusitis has been proposed (see Patent Document 1). In case of an antibacterial agent-containing eye drops, exacerbation of corneal permeability of a drug, and increase in an amount of penetration into an aqueous humor become an index of preparation design.

However, generally, a drug applied to eyes is hardly penetrated into eyes due to dilution with a tear fluid and the barrier function of a cornea. For this reason, it is necessary to improve intraocular penetration of a drug, and design a preparation so as to enhance the effect of a drug.

Ophthalmic liquid preparations containing quinolonecarboxylic acid antibacterial agent are variously known and, for example, Patent Document 2 discloses an eye drop solution stable to full daylight, which contains hyaluronic acid or a salt thereof in an isotonic solution containing a quinolonecarboxylic acid synthetic antibacterial substance at pH 6.0 to 7.0. Patent Document 3 relates to an aqueous composition of quinolonecarboxylic acid, and describes a stable aqueous composition containing lomefloxacin, which is obtained by adding a polyhydric alcohol, or boric acid to lomefloxacin to make it isotonic and, further, adjusting a pH of 3 to 6.5. As the polyhydric alcohol, glycerin is exemplified. This aqueous composition includes eye drops, and solubility data of lomefloxacin upon use of glycerin for sodium chloride are also described. Patent Document 4 relates to an ophthalmic aqueous liquid preparation containing gatifloxacin and sodium edetate, and also discloses a method for raising corneal permeability of gatifloxacin, a method for preventing crystal precipitation of gatifloxacin, and a method for preventing coloration of gatifloxacin. In addition, as optional additives, sodium hyaluronate and glycerin are exemplified.
Patent Document 1: JP 8-9597 B
Patent Document 2: JP 2002-37746 A
Patent Document 3: JP 63-174930 A
Patent Document 4: WO 00/10570 A

### Disclosure of the Invention

### Problem to be Solved by the Invention

An object of the present invention is to provide an aqueous liquid preparation excellent in intraocular penetration, that is, retention in a tear fluid, and penetration into an aqueous humor and a conjunctiva of gatifloxacin.

### Means for Solving the Problem

In order to attain the object, the present inventor has intensively studied and, as a result, has found out that, by formulating hyaluronic acid and a polyhydric alcohol in a gatifloxacin-containing aqueous liquid preparation, intraocular penetration of gatifloxacin can be improved, resulting in completion of the present invention.

That is, the present invention provides:
(1) An aqueous liquid preparation including (A) gatifloxacin, its pharmacologically acceptable salt or a hydrate thereof, (B) hyaluronic acid or its pharmacologically acceptable salt, and (C) a polyhydric alcohol;
(2) The aqueous liquid preparation according to the above (1), wherein the concentration of gatifloxacin is 0.1 to 1.0 w/v%;
(3) The aqueous liquid preparation according to the above (1), wherein the concentration of gatifloxacin is 0.2 to 0.8 w/v%;
(4) The aqueous liquid preparation according to the above (1), wherein the concentration of hyaluronic acid is 0.01 to 1.0 w/v%;
(5) The aqueous liquid preparation according to the above (1), wherein the concentration of hyaluronic acid is 0.02 to 0.8 w/v%;
(6) The aqueous liquid preparation according to the above (1), wherein the concentration of hyaluronic acid is 0.05 to 0.5 w/v%;
(7) The aqueous liquid preparation according to the above (1), wherein the concentration of the polyhydric alcohol is 0.1 to 10 w/v%;
(8) The aqueous liquid preparation according to the above (1), wherein concentration of the polyhydric alcohol is 1.0 to 5.0 w/v%;
(9) The aqueous liquid preparation according to the above (1), wherein the polyhydric alcohol is at least one compound selected from glycerin propylene glycol and mannitol;
(10) The aqueous liquid preparation according to the above (1), which is ophthalmic;
(11) The aqueous liquid preparation according to the above (1), which is an eye drop,
(12) A method for improving intraocular penetration of gatifloxacin, which comprises incorporating hyaluronic acid or its pharmacologically acceptable salt, and a polyhydric alcohol into gatifloxacin, its pharmacologically acceptable salt or a hydrate thereof;
(13) Use of hyaluronic acid or its pharmacologically acceptable salt and a polyhydric alcohol for manufacturing an ophthalmic aqueous liquid preparation having intraocular penetration of gatifloxacin containing gatifloxacin, its pharmacologically acceptable salt, or a hydrate thereof; and the like.

### Effect of the Invention

According to the present invention, intraocular penetration of gatifloxacin is improved by adding hyaluronic acid or its pharmacologically acceptable salt and a polyhydric alcohol to an aqueous liquid preparation containing gatifloxacin, its pharmacologically acceptable salt, or a hydrate thereof,.

### Brief Description of Drawings

Fig. 1 is a graph showing results of measurement of gatifloxacin concentration in a tear fluid in Test Example 1.
Fig. 2 is a graph showing results of measurement of gatifloxacin concentration in an aqueous humor in Test Example 2.
Fig. 3 is a graph showing results of measurement of gatifloxacin concentration in a conjunctiva in Test Example 2.

### Best Mode for Carrying Out the Invention

In the present specification, improvement in intraocular penetration means improvement in an amount of penetration of a drug into a tear fluid, an aqueous humor or a conjunctiva.

The present invention uses gatifloxacin, its pharmacologically acceptable salt, or a hydrate thereof as an active ingredient. Examples of the pharmacologically acceptable salt of gatifloxacin include salts with inorganic acids such as hydrochloric acid, sulfuric acid and phosphoric acid, salts with organic acids such as methanesulfonic acid, lactic acid, oxalic acid and acetic acid, and salts of sodium, potassium, magnesium, calcium, aluminum, cerium, chromium, cobalt, copper, iron, zinc, platinum, silver and the like. Examples of the hydrate include 2/5, 1/2, 3/2, and 5 hydrates. The amount of gatifloxacin, its pharmacologically acceptable salt, or a hydrate thereof to be incorporated into the aqueous liquid preparation of the present invention is varied depending on a degree of infection to be subjected, but is usually 0.1 to 1.0 w/v%, and preferably 0.2 to 0.8 w/v% as free gatifloxacin based on the total amount of the aqueous liquid preparation.

In the present invention, examples of the pharmacologically acceptable salt of hyaluronic acid include a sodium salt, a potassium salt, a calcium salt, a magnesium salt and a basic amino acid salt.

The average molecular weight of hyaluronic acid used in the present invention is usually 300000 to 8000000, preferably 500000 to 3000000.

The amount of hyaluronic acid or its pharmacologically acceptable salt is usually 0.01 to 1.0 w/v%, preferably 0.02 to 0.8 w/v %, and further preferably 0.05 to 0.5 w/v% as hyaluronic acid based on the total amount of the aqueous liquid preparation.

Examples of the polyhydric alcohol include glycerin, propylene glycol, mannitol, polyethylene glycol, xylitol, and polyvinyl alcohol, preferably glycerin, propylene glycol, and mannitol, and one or more of them can be used. Particularly preferable is glycerin.

The amount of the polyhydric alcohol to be incorporated is usually 0.1 to 10 w/v%, preferably 1.0 to 5.0 w/v% based on the total amount of the aqueous liquid preparation.

The aqueous liquid preparation of the present invention can be administered orally or parenterally. Examples of a dosage form include oral dosage forms such as syrups, and parenteral dosage forms such as solution-like injectable preparations, external preparations such as eye drops, etc., and the aqueous liquid preparation is preferably used in a form for ocular topical administration and particularly preferably eye drops.

The pH of the aqueous liquid preparation of the present invention is usually 5 to 8, preferably 5.5 to 7.5, further preferably 5.8 to 7.2, and particularly preferably 6 to 7.

Further, if necessary, the aqueous liquid preparation of the present invention may appropriately contain isotonicities (e.g. sodium chloride, potassium chloride, boric acid, glucose, etc.), buffers (e.g. phosphate buffer, acetate buffer, borate buffer, citrate buffer, glutamic acid, ε-aminocaproic acid, etc.), preservatives (benzalkonium chloride, benzethonium chloride, chlorohexidine gluconate, chlorobutanol, benzyl alcohol, sodium dehydroacetate, paraoxybenzoic acid esters, etc.), thickeners (methylcellulose, hydroxy ethylcellulose, hydroxypropylmethylcellulose, carboxymethylcellulose, carboxyvinyl polymer, polyvinyl alcohol, polyvinylpyrrolidone, macrogol, etc.), pH adjusting agents (hydrochloric acid, sodium hydroxide, acetic acid, phosphoric acid, etc.), stabilizers (sodium edetate, citric acid, etc.) and the like.

The aqueous liquid preparation of the present invention may be produced by a per se known method, and can be produced, for example, by the method described in 15^{th} revision Japanese Pharmacopoeia, Preparation General Rule, Eye drops or Liquid Preparations.

The aqueous liquid preparation of the present invention, for example, eye drops have antibacterial activity, and can be applied one drop to each eye 1 to 3 times a day for preventing or treating blepharitis, hordeolum, dacryocytitis, conjunctivitis, inflammation of the tarsal gland, keratitis, cornea ulcer, post-operation infectious disease, and the like. For otitis externa and tympanitis, the preparation can be usually applied 6 to 10 drops into each ear 1 to 2 times a day. In addition, for sinusitis, usually, the preparation can be spray-inhaled 2 to 4 ml every other day three times per week, or the preparation can be injected into maxillary sinus 1 ml per week. In this regard, the number of applications can be appropriately increased or decreased depending on a degree of a particular symptom.

### Examples

The following Examples and Test Examples will further illustrate the present invention in detail, but the present invention is not limited thereto.

### Example 1 and Comparative Examples 1 to 3

### Test Example 1

Effect of hyaluronic acid and glycerin on concentration of gatifloxacin in tear fluid

### 1. Experimental material

According to the formulations shown in Table 1, gatifloxacin-containing eye drops were prepared by a conventional method.

**Table 1**

| Ingredient | Example 1 | Comparative Example 1 | Comparative Example 2 | Comparative Example 3 |
|---|---|---|---|---|
| Gatifloxacin 3/2 hydrate | 0.32 g | 0.32 g | 0.32 g | 0.32 g |
| Sodium hyaluronate | 0.125 g | - | 0.125 g | - |
| Sodium chloride | - | 0.9 g | 0.9 g | - |
| Concentrated glycerin | 2.5 g | - | - | 2.5 g |
| Hydrochloric acid/sodium hydroxide | adequate amount | adequate amount | adequate amount | adequate amount |
| Distilled water | adequate amount | adequate amount | adequate amount | adequate amount |
| Total amount | 100 mL | 100 mL | 100 mL | 100 mL |
| pH | 7.0 | 7.0 | 7.0 | 7.0 |

| | | | | |
|---|---|---|---|---|
| Sodium hyaluronate having an average molecular weight of 1.2 million manufactured by Seikagaku Corporation was used. | | | | |

### 2. Experimental method

Each 50 µL of gatifloxacin eye drops of Table 1 was applied to eyes of a Japanese white male domestic rabbit having a weight of about 2 kg. After 5, 15, 30, and 60 minutes of application to eyes, the tear fluid was collected using a capillary (2 µL). The weight of the collected tear fluid was measured, and diluted with 300 µL of a diluent (mixed solution of 0.1% aqueous phosphoric acid solution and acetonitrile (4 : 1)), which was used as a sample solution. Regarding 20 µL of the sample solution, concentration of gatifloxacin was measured by a HPLC method.

### HPLC conditions

Detector: Ultraviolet spectrophotometer (measurement wavelength: 280 nm)
Column: Inertsil ODS-II (manufactured by GL Sciences Inc.) Column temperature: 40 °C
Mobile phase: phosphoric acid was added to a mixed solution of acetonitrile, water and triethylamine (18 : 81 : 1) to adjust a pH to 4.5.
Injection amount: 20 µL
Flow rate: 0.8 mL/min

### 3. Results

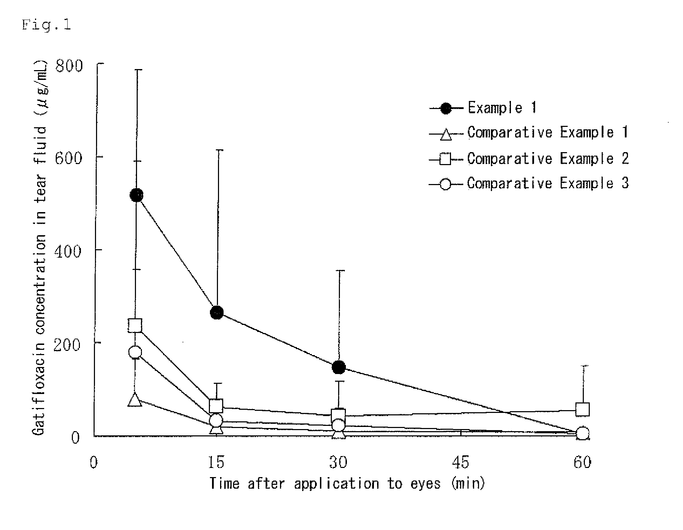

Results are shown in Table 2 and Fig. 1.

**Table 2**

| Concentration of gatifloxacin in tear fluid | | | | |
|---|---|---|---|---|
| Time | Example 1 | Comparative Example 1 | Comparative Example 2 | Comparative Example 3 |
| | (n=6) | (n=6) | (n=6) | (n=6) |
| After 5 min | 516.41±270.27 | 81.15±83.30 | 237.09±353.43 | 180±177 |
| After 15 min | 264.39±348.38 | 20.30±14.70 | 62.63±49.63 | 32±45 |
| After 30 min | 147.50±207.66 | 8.99±8.96 | 42.13±75.00 | 23±37 |
| After 60 min | 5.62±4.04 | 8.66±5.71 | 53.87±96.61 | 4±6 |

| | | | | |
|---|---|---|---|---|
| Each value indicates average±standard deviation (µg/mL). | | | | |

From these results, it has been found that, in Example 1 of the present invention which contains sodium hyaluronate and glycerin, penetration of gatifloxacin into a tear fluid is synergistically improved as compared with Comparative Example 2 containing sodium hyaluronate, and Comparative Example 3 containing glycerin.

### Example 2 and Comparative Example 4

### Test Example 2

Effect of hyaluronic acid on concentration of gatifloxacin in aqueous humor and conjunctiva

### 1. Test method

According to the formulations of Table 3, gatifloxacin eye drops were prepared, and each 50 µL was applied to eyes of a Japanese white male domestic rabbit having a weight of about 2.5 kg. One hour after application to eyes, an excessive amount of 5% pentobarbital sodium was administered to euthanize the animal. After an anterior eye was washed with physiological saline, an aqueous humor and a conjunctiva were collected. The aqueous humor was collected with a syringe with 27G injection needle, and the conjunctiva was directly cut with scissors. The aqueous humor was filtered with a 0.22 µm membrane filter, which was used as a sample solution. The conjunctiva, after measurement of the weight, was finely cut by adding 5 mL of acetonitrile, followed by shaking (200 rpm × 20 min) and centrifugation (2000 rpm × 10 min). Four milliliter of this supernatant was taken, evaporated to dryness under reduced pressure, and then dissolved in 0.5 mL of a mobile phase for HPLC, and filtered with a filter (0.22 µm), which was used as a sample solution. Regarding 50 µL of each sample solution, concentration of gatifloxacin was measured by the HPLC method.

**Table 3**

| Ingredient | Example 2 | Comparative Example 4 |
|---|---|---|
| Gatifloxacin 3/2 hydrate | 0.32 g | 0.32 g |
| Sodium hyaluronate | 0.2 g | - |
| Concentrated glycerin | 2.5 g | - |
| Sodium chloride | - | 0.86 g |
| Hydrochloric acid | adequate amount | adequate amount |
| Purified water | adequate amount | adequate amount |
| Total amount | 100 mL | 100 ml |
| pH | 6.0 | 6.0 |

| | | |
|---|---|---|
| Sodium hyaluronate having an average molecular weight of 1.2 million manufactured by Seikagaku Corporation was used. | | |

HPLC conditions
HPLC analysis conditions
Detector: Ultraviolet visible spectrophotometer
(measurement wavelength: 280 nm)
Column: Inertsil ODS-3 4.6 mmφ × 150 mm, 5 µm (GL Sciences Inc.)
Guard column: Inertsil ODS-3 4.6 mmφ × 5 mm (GL Sciences Inc.)
Column temperature: 40°C
Mobile phase: Phosphoric acid was added to a mixed solution of acetonitrile, distilled water and triethylamine (18 : 81 : 1) to adjust a pH to 4.5.
Flow rate: 0.8 mL/min
Injection amount: 50 µL
Setting temperature of sample cooler: 4°C

### 3. Results

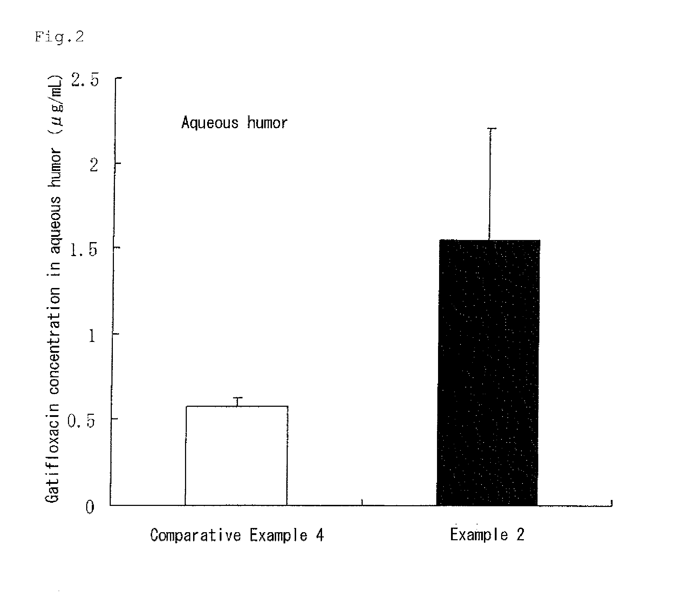

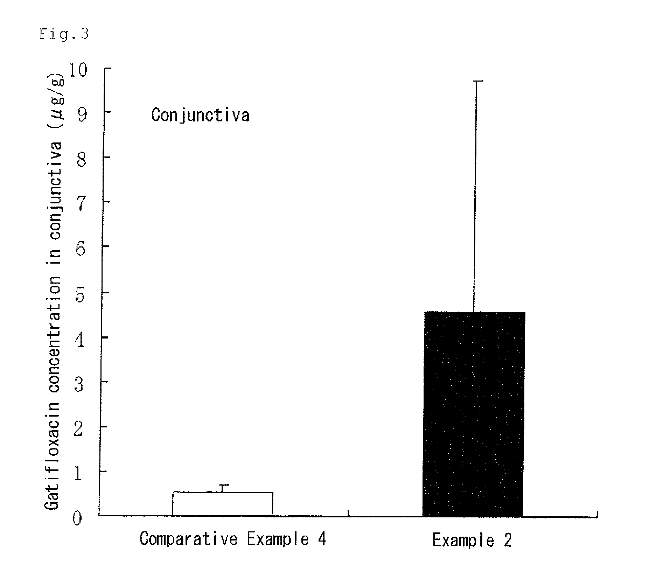

Results are shown in Table 4, Fig. 2 and Fig. 3.

**Table 4**

| Concentration of gatifloxacin in aqueous humor and conjunctiva 1 hour after application to eyes | | |
|---|---|---|
| | Concentration of gatifloxacin in aqueous humor (µg/mL) | Concentration of gatifloxacin in conjunctiva (µg/g) |
| Example 2 (n=3) | 1.545±0.659 | 4.570±5.166 |
| Comparative Example 4 (n=3) | 0.574±0.049 | 0.538±0.156 |

| | | |
|---|---|---|
| Each value indicates average±standard deviation. Examples 3 to 8 | | |

According to the formulation of Table 5, gatifloxacin-containing eye drops were prepared by a conventional method.

**Table 5**

| Formulation (g/100 mL) | Example 3 | Example 4 | Example 5 | Example 6 | Example 7 | Example 8 |
|---|---|---|---|---|---|---|
| Gatifloxacin 3/2 hydrate | 0.2 | 0.3 | 0.3 | 0.5 | 0.5 | 0.8 |
| Sodium hyaluronate | 0.1 | 0.1 | 0.2 | 0.2 | 0.3 | 0.2 |
| Concentrated glycerin | - | 2.5 | - | - | 2.5 | - |
| Propylene glycol | - | - | 2.0 | - | - | - |
| Mannitol | 2.5 | - | - | 5.0 | - | 5.0 |
| Disodium edetate | - | 0.02 | - | - | 0.02 | - |
| Sodium dihydrogen phosphate | - | - | 0.1 | - | - | - |
| Benzalkonium chloride | - | 0.005 | - | - | - | 0.005 |
| Methyl paraoxybenzoate | - | - | - | 0.026 | - | - |
| Propyl paraoxybenzoate | - | - | - | 0.014 | - | - |
| Boric acid | 0.8 | - | - | - | - | - |
| Hydrochloric acid/sodium hydroxide | adequate amount | adequate amount | adequate amount | adequate amount | adequate amount | adequate amount |
| Sterile purified water | adequate amount | adequate amount | adequate amount | adequate amount | adequate amount | adequate amount |
| Total amount | 100 mL | 100 ml | 100 mL | 100 ml | 100 mL | 100 ml |
| pH | 7.0 | 6.5 | 6.0 | 6.0 | 6.0 | 6.0 |

| | | | | | | |
|---|---|---|---|---|---|---|
| Sodium hyaluronate having an average molecular weight of 1.2 million manufactured by Seikagaku Corporation was used. | | | | | | |

### Industrial Applicability

As described hereinabove, according to the present invention, it is possible to provide a gatifloxacin-containing ophthalmic aqueous liquid preparation having improved intraocular penetrating of gatifloxacin by incorporating hyaluronic acid and a polyhydric alcohol thereinto.

## Claims

1. An aqueous liquid preparation comprising (A) gatifloxacin, its pharmacologically acceptable salt or a hydrate thereof, (B) hyaluronic acid or its pharmacologically acceptable salt, and (C) a polyhydric alcohol.

2. The aqueous liquid preparation according to claim 1, wherein the concentration of gatifloxacin is 0.1 to 1.0 w/v%.

3. The aqueous liquid preparation according to claim 1, wherein the concentration of hyaluronic acid is 0.01 to 1.0 w/v%.

4. The aqueous liquid preparation according to claim 1, wherein the concentration of the polyhydric alcohol is 0.1 to 10 w/v%.

5. The aqueous liquid preparation according to claim 1, wherein the polyhydric alcohol is at least one compound selected from glycerin, propylene glycol and mannitol.

6. The aqueous liquid preparation according to claim 1, which is an eye drop.

7. A method for improving intraocular penetration of gatifloxacin, which comprises incorporating hyaluronic acid or its pharmacologically acceptable salt, and a polyhydric alcohol into gatifloxacin, its pharmacologically acceptable salt or a hydrate thereof.
